Europäisches Patentamt

European Patent Office

Office européen des brevets

(19)

(11) Publication number: **0 080 357**
A1

(12)

# EUROPEAN PATENT APPLICATION

(21) Application number: **82306205.4**

(22) Date of filing: **22.11.82**

(51) Int. Cl.³: **C 07 H 21/04, A 61 K 31/71**

(30) Priority: **23.11.81 US 324241**
**23.11.81 US 324243**

(43) Date of publication of application: **01.06.83**
**Bulletin 83/22**

(84) Designated Contracting States: **AT BE CH DE FR GB IT LI LU NL SE**

(71) Applicant: **ELI LILLY AND COMPANY, 307, East McCarty Street, Indianapolis Indiana 46285 (US)**

(72) Inventor: **Douthart, Richard James, 209 Enterprise, Richland Washington 99352 (US)**
Inventor: **Kleinschmidt, Walter John, 9732 Trilobi Drive, Indianapolis Indiana 46236 (US)**
Inventor: **Schultz, Richard Micheal, 8700 Shelby Street, Indianapolis Indiana 46227 (US)**

(74) Representative: **Hudson, Christopher Mark et al, Erl Wood Manor, Windlesham Surrey GU20, 6PH (GB)**

(54) Composition for treating tumor cells.

(57) There is described a composition for use in inhibiting the proliferation and migration of tumor cells which comprises a natural or synthetic double stranded RNA and an aminoglycoside having at least 5 ionizable nitrogens, said aminoglycoside and double stranded RNA being present in a molar ratio, aminoglycoside to double stranded RNA phosphorus, of at least 1:1.

EP 0 080 357 A1

## COMPOSITION FOR TREATING TUMOR CELLS

This invention relates to complexes of certain aminoglycosides and natural or synthetic double stranded RNA which are useful in inhibiting the proliferation and migration of tumor cells.

The complexes comprise a natural or synthetic double stranded RNA (dsRNA) and an aminoglycoside having at least 5 ionizable nitrogens, said aminoglycoside and double stranded RNA being present in a molar ratio, aminoglycoside to double stranded RNA phosphorus, of at least 1:1.

The complexes are prepared by mixing dsRNA and aminoglycoside in amounts to obtain a complex having the desired molar ratio.

Interferon is a naturally-occurring antiviral protein produced by most cells upon stimulation by virus infection or other substances recognized as interferon "inducers". The potential of interferon as a broad spectrum antiviral and/or anti-tumor agent has long been recognized.

Double stranded RNA (dsRNA), whether of synthetic, e.g., polyriboinosinic-polyribocytidilic acid (poly I:poly C), or natural origin, is recognized as an excellent inducer of interferon. Unfortunately, the usefulness of natural or synthetic dsRNA is greatly limited due to its rapid degradation by nucleases present in the sera of various animal species, particularly primates. More significantly, the enzymatic degradation of dsRNA is especially apparent in human serum; see, for example, R.J. Douthart and S.G. Burgett, Biochem. and Biophys. Res. Comm. 84, (1978), 809-815.

It is essential, therefore, if dsRNA is to be useful as an inducer of interferon in primates, in particular, humans, that its rapid enzymatic degradation be prevented or substantially diminished.

Attempts have been made to promote the interferon-inducing activity of dsRNA, for example, by stabilizing it against enzymatic degradation by a variety of approaches.

W.E. Houston, C.L. Crabbs, E.L. Stephen, and H.B. Levy, Infection and Immunity 14, (1976), 318-319, report stabilization of poly I:poly C against enzymatic degradation using poly-L-lysine and carboxymethylcellulose.

A. Billiau, C.E. Buckler, F. Dianzani, C. Uhlendorf, and S. Baron, Ann. N.Y. Acad. Sci., 173 (1970) 657-667, report that stimulation of the interferon mechanism in tissue culture by poly I:poly C can be enhanced by addition of substances such as neomycin, streptomycin, diethylaminoethyl dextran (DEAE-dextran), methylated albumin, protamine, histone, and colistin. Neomycin and protamine, each when administered to mice in conjunction with poly I:poly C, were shown to have no effect on the yield of circulating interferon. DEAE-dextran, on the other hand, showed a definite enhancement of the interferon response from separately-administered poly I:poly C.

G.P. Lampson, A.A. Tytell, A.K. Field, M.M. Nemes, and M.R. Hilleman, Proc. Soc. Exp. Biol. and Med., 132, (1969) 212-218, measured the effect of several polyamines used in conjunction with poly I:poly C. Of ten polyamines tested, five demonstrated some effect toward the stabilization of poly I:poly C in the presence of RNase. Most of the testing was carried out using neomycin with the stated conclusion that the "effect of neomycin was entirely limited to in vitro activity in one particular kind of cell and showed no potential for practical utilization in human and animal application either in potentiation of poly I:C activity or in reduction of its toxicity." (page 217).

J.J. Nordlund, S.M. Wolff, and H.B. Levy, Proc. Soc. Exp. Biol. and Med., 133, (1970) 439-444, suggest that human plasma is capable of rapid enzymatic degradation of poly I:poly C in contrast, e.g., to rabbit serum. They further report the elimination by neomycin of the destructive capacity of dilute (15%) human plasma.

The literature also describes the use of dsRNA as an anti-tumor agent. Heretofore, it has been recognized that synthetic dsRNA inhibits tumor growth in mice [H. B. Levy, L. W. Law, and A. S. Robson, Proc. Nat. Acad. Sci., 62, 357-361 (1969)]; is active in the treatment of leukemic mice [L. D. Zeleznick and B. K. Bhuyan, Proc. Soc. Exp. Biol. Med., 130, 126-128 (1969)]; and inhibits chemically-induced tumorigenesis in mouse skin [H. V. Gelboin and H. B. Levy, Science, 167, 205-207 (1970)].

It has now been discovered that complexes of certain aminoglycosides and natural or synthetic dsRNA exhibit the highly advantageous properties of (1) ability to activate peritoneal macrophages; and (2) enhancement of the activity of dsRNA against tumor systems, including highly refractory tumors such as the Madison lung tumor. These complexes exhibit a potentiation of the tumor effect relative to dsRNA alone. More significantly, this potentiating effect is wholly unexpected since it apparently is entirely unrelated to any inhibition to nuclease-mediated degradation of dsRNA. This is demonstrated by the fact that the potentiation occurs even in species having low levels of dsRNase, i.e., in those in which there is no demonstrable difference between the peak interferon titers produced by dsRNA alone and that produced by a complex of the aminoglycosides and dsRNA. The dsRNA used in the complexes can be synthetic, such as, for example, poly I:poly C, poly A:poly U, poly G:poly C, and the like. Likewise, and preferably, the dsRNA can be of natural origin. Among the specific sources of natural dsRNA are virus particles found in certain strains of <u>Penicillium</u> <u>chrysogenum</u>, <u>Penicillium</u> <u>funiculosum</u>, <u>Penicillium</u> <u>stoloniferum</u>, <u>Aspergillus</u> <u>niger</u>, <u>Aspergillus</u> <u>foetidus</u>, <u>Ø6 bacteriophage</u>, and the like.

A preferred strain useful in the production of dsRNA is <u>Penicillium</u> <u>chrysogenum</u>. Methods for producing and isolating dsRNA are well recognized in the literature, see, e.g., U.S. Patent No. 3,597,318; and U.S. Patent No. 3,582,469.

The complexes useful in inhibiting the proliferation and migration of tumor cells comprise dsRNA and at least one aminoglycoside. The aminoglycosides that are used have at least 5 ionizable nitrogens in their structure. Examples of such aminoglycosides including the number of ionizable nitrogens are neomycin (6), tobramycin (5), kanamycin B (5), gentamycin (5), apramycin (5), and the like. Preferably, the aminoglycoside is one having 5 ionizable nitrogens, and, of these, tobramycin and apramycin are highly preferred with tobramycin being the most preferred.

The relative amounts of dsRNA and aminoglycoside present in the complexes are defined in terms of the B/P ratios in which B represents moles of aminoglycoside and P represents moles of dsRNA phosphorus. Although there is no upper limit for the B/P ratio, in order to achieve the desired effectiveness, the ratio must be at least about 1:1.

The preferred B/P ratio is from about 12:1 to about 1:1, and most preferably, is from about 9:1 to about 6:1.

The following represents a general procedure for producing a complex. Lyophilized dsRNA is dissolved in 0.02$\underline{M}$ Tris 0.1$\underline{M}$ NaCl pH 7.0 buffer. The solution is dialyzed overnight against the same buffer system. The procedure uses baked glassware throughout, and all buffers are filtered through a Nalgene 45 micron filter for sterility. Pyrogen-free double distilled water is used for all solutions to minimize any possibility of endotoxin contamination.

The concentration of the dsRNA solution is determined from its UV spectrum on the following basis:

$$44.7 \times OD_{260} = \text{micrograms dsRNA/ml.}$$

For example, for Penicillium chrysogenum dsRNA, the moles of RNA phosphorus are determined from the optical density (OD) at 260 nm using an extinction coefficient of 7200. The extinction coefficients of other natural and synthetic double stranded RNA's can be obtained from the literature or determined using standard procedures and this coefficient then used in determining the molar concentration of dsRNA phosphorus.

If appropriate, the dsRNA solution can be diluted with pyrogen-free buffer to facilitate formation of a complex having the desired B/P ratio.

A stock solution of the aminoglycoside is prepared using the same buffer used for the dsRNA solution. The molar concentration of the aminoglycoside as free base is calculated. This can be determined (a) from its known potency value or (b) from its chemical structure. The pH of the aminoglycoside stock solution then is carefully adjusted to 7.0 using $1\underline{N}$ HCl.

Aliquots of the dsRNA and aminoglycoside solutions are mixed in amounts to obtain a complex having the desired B/P ratio.

The complex can be used as is in which case it is incubated for approximately one hour before use. Alternatively, the complex can be isolated and reconstituted in another suitable carrier. The complex is stable in any physiologically acceptable carrier, generally in combination with a supporting monovalent ion concentration of about $0.1\underline{M}$ buffered to a pH of about 6.8 to about 7.2. Physiological saline in bicarbonate or phosphate buffer is a preferred carrier.

The complexes have wide applicability. Since dsRNA is rapidly destroyed in human serum due to the presence of degrading RNase, dsRNA in that environ has little or no biological activity. These complexes, however, exhibit excellent interferon-inducing and

0080357

macrophage-activation properties in conjunction with their anti-tumor activity, even in the presence of dsRNA-degrading RNase. Thus, these complexes are particularly useful in humans in the presence of human serum RNase.

The complexes exhibit a potentiation of the tumor effect relative to dsRNA alone. This potentiation is wholly unexpected since it exists even in the absence of human serum RNase which is shown to degrade dsRNA but not the complexes used in this invention. Desirably, these complexes are particularly useful in adjuvant cancer therapy to inhibit metastatic cancer development.

The complexes can be prepared in a variety of pharmaceutical compositions and formulations and can be administered by a variety of conventional routes, such as intramuscular, intravenous, subcutaneous, topical, and intraperitoneal.

The compositions can be administered parenterally or intraperitoneally. The pharmaceutical forms suitable for injection include sterile aqueous solutions or dispersions and sterile powders for reconstitution into sterile injectible solutions or dispersions. The carrier can be a solvent or dispersing medium containing, for example, water, ethanol, polyol (for example glycerol, propylene glycol, liquid polyethylene glycol, and the like), suitable mixtures thereof, and vegetable oils. Proper fluidity can be maintained, for example, by the use of a coating such as lecithin, by the maintenance of the required particle size in the case of dispersion and by the use of surfactants.  Prevention of the action of microorganisms can be ensured by various antibacterial and antifungal agents, for example, parabens, chlorobutanol, phenol, sorbic acid, and the like.  In many cases, it will be desirable to include isotonic agents, for example, sugars, sodium chloride, and the like.  Prolonged absorption of the injectable pharmaceutical form can be brought about by the use of agents delaying absorption, for example, aluminum monostearate and gelatin.

Sterile injectable solutions can be prepared by incorporating the complex in the required amount of the appropriate solvent with various of the other ingredients, as desired.

For more effective distribution, the complex can be incorporated into slow release or targeted delivery systems such as polymer matrices, liposomes, and microspheres.

As noted, the complexes also can be used topically, for example, for the treatment of skin carcinomas.  Thus, the complexes can be formulated as ointments, creams, lotions, and the like.

In formulating an ointment, the complex, for example, is finely dispersed in paraffin.  Liquid paraffin, hard paraffin, and wool fat may be included in the ointment base.  If a water-miscible ointment base is desired, a polyethylene glycol may be included.

The complex may also be formulated as a cream, which may be an oil-in-water type or a water-in-oil type.  Suitable emulsifying agents for the former are sodium, potassium, ammonium and triethanol soaps; polysorbates; and cationic, anionic, and nonionic emulsifying waxes. Suitable emulsifying agents
for the latter type are calcium soaps, wool fat, wool
alcohols, beeswax, and certain sorbitan esters. A
preservative usually is desirable in a cream, particularly an aqueous cream. Examples of suitable preservatives, alone or in combination, are chlorocresols,
p-hydroxybenzoates, and the like.

The complex may be formulated as a lotion by
dissolving or dispersing it in an aqueous or oily base.
Ethanol and/or glycerin may be included in the aqueous
base. Examples of suitable oil bases are castor oil,
vegetable oils, and the like. A suitable preservative
may be incorporated in the formulation.

As used herein, the term "pharmaceutically
acceptable carrier" includes any and all solvents,
dispersion media, coatings, antibacterial and antifungal agents, isotonic agents, absorption delaying
agents, and the like. The use of such media and agents
for pharmaceutically active substances is well known in
the art. Except insofar as any conventional media or
agent is incompatible with the compositions, its use in
the therapeutic formulation is contemplated. Supplementary active ingredients can also be incorporated
into the pharmaceutical formulations.

It is especially advantageous to formulate
parenteral compositions in dosage unit form for ease of
administration and uniformity of dosage. Dosage unit
form as used herein refers to a physically discrete
unit suited as unitary dosages for the subject to be
treated. Each unit contains a predetermined quantity
of the complex calculated to produce the desired therapeutic effect in association with the pharmaceutically acceptable carrier. The specific dosage unit form is dictated by and directly dependent upon (a) the unique characteristics of the particular composition and (b) the particular therapeutic effect to be achieved.

In general, the compositions can be administered to a host in an effective amount as an adjunct to tumor chemotherapy, tumor radiation therapy, and/or tumor excision. The composition can be administered at the same time as such therapy or within an appropriate time prior to or subsequent to such therapy.

As indicated, the compositions are administered parenterally. Such administration generally will be an amount ranging, for example, from about 5 µg. to about 500 µg. dsRNA per kg. of body weight, and preferably, from about 5 µg. to about 100 µg. per kg. of body weight, and, most preferably, from about 20 µg. to about 60 µg. per kg. of body weight.

The following examples are provided to illustrate the instant invention. They are not intended to be limiting upon the scope thereof.

## Example 1

Tobramycin-Penicillium Chrysogenum dsRNA (PCMdsRNA) Complex, B/P = 7.

The absorption spectra of the PCMdsRNA stock was recorded. The concentration of RNA phosphorus was determined using an εp value of 7200. To 4 ml. of a solution of $1.24 \times 10^{-4}$ mM phosphorus ($OD_{260}$ = 0.895) PCMdsRNA in 0.02M Tris, 0.1M NaCl, pH 7.0 were added 4 ml. of a solution containing 0.436 mg./ml. tobramycin in the same buffer brought to pH 7.0 with 0.1$\underline{N}$ HCl. The potency of the tobramycin sample was 0.93 mg. free base/mg. sample. The final concentrations were 4.35 x $10^{-4}$ mM tobramycin as free base and 6.21 x $10^{-5}$ mM PCMdsRNA phosphorus which is a B/P of 7.0. The solution was allowed to incubate for 1 hour at room temperature before use.

### Example 2

Tobramycin-Penicillium Chrysogenum dsRNA (PCMdsRNA) Complex, B/$\overline{P}$ = 2

To 4 ml. of a solution of 1.24 x $10^{-4}$ mM phosphorus ($OD_{260}$ = 0.895) PCMdsRNA in 0.02$\underline{M}$ Tris, 0.1$\underline{M}$ NaCl, pH 7.0 were added 4 ml. of a solution containing 0.125 mg./ml. tobramycin in the same buffer brought to a pH of 7.0 with 0.1$\underline{N}$ HCl. The potency of the tobramycin samples was 0.932 mg. free base/mg. sample. The final concentrations were 0.124 x $10^{-4}$ mM moles tobramycin as free base and 6.21 x $10^{-5}$ mM PCMdsRNA phosphorus which is a B/P of 2.0. The solution was allowed to incubate for 1 hour at room temperature before use.

### Example 3

Tobramycin - Poly I-Poly C Complex, B/P = 7.

To 10 mg. of lyophilized salt-free Poly I-Poly C (P. L. Biochemicals, Lot 547261) were added 10 ml. of 0.02$\underline{M}$ Tris, 0.1$\underline{M}$ NaCl, pH 7.0 buffer. The samples was equilibrated for 1 hour at 50 C. and allowed to cool slowly overnight to room temperature. An εp of 5200 was used to determine Poly I-Poly C phosphorus concentration.

To 4 ml. of the Poly I-Poly C duplex solution ($OD_{260}$ = 0.64, concentration of phosphorus = 1.23 x $10^{-4}$ mM) were added 4 ml. of a solution containing 0.436 mg./ml. of tobramycin in the same buffer brought to a pH of 7.0 with 0.1N HCl. The potency of the tobramycin was 0.93 mg. free base/mg. sample. The final concentrations were 4.35 x $10^{-4}$ mM tobramycin as free base and 6.15 x $10^{-5}$ mM Poly I-Poly C phosphorus which is a P/B of 7.0. The solution was allowed to incubate for 1 hour at room temperature.

Example 4

Streptomycin-Penicillium Chrysogenum dsRNA (PCMdsRNA) Complex, B/P = 7.0

To 4.0 ml. of a solution of 1.24 x $10^{-4}$ mM phosphorus ($OD_{260}$ = 0.895) PCMdsRNA in .02M Tris, 0.10M NaCl, pH 7.0 buffer, were added 4.0 ml. of a solution containing 0.844 mg./ml. of streptomycin in the same buffer brought to pH 7.0 with 0.1N HCl. The potency of the streptomycin sample was 0.750 mg. free base/mg. sample.

The final concentrations were 4.35 x $10^{-4}$ mM streptomycin as free base and 6.22 x $10^{-4}$ mM PCMdsRNA phosphorus which is a B/P of 7.0. The solution was incubated for 1 hour at room temperature to ensure complex formation before use.

## Example 5

Neomycin-Penicillium Chrysogenum dsRNA (PCMdsRNA)
Complex, B/P = 7.0

To 4.0 ml of a solution of $1.24 \times 10^{-4}$ mM
phosphorus ($OD_{260} = 0.895$) PCMdsRNA in .02M Tris, 0.1M
NaCl, pH 7.0 buffer were added 4.0 ml. of a solution
containing 0.77 mg./ml. of neomycin sulfate in the same
buffer brought to pH 7.0 with 0.1N HCl. The potency of
the neomycin sulfate sample was 0.70 mg. free base/mg.
sample.

The final concentrations were $4.35 \times 10^{-4}$ mM
neomycin as free base and 6.215 $\times 10^{-4}$ mM PCMdsRNA
phosphorus which is a B/P of 7.0. The solution was
incubated 1 hour at room temperature to ensure complex
formation before use.

## Example 6

Apramycin-Penicillium Chrysogenum dsRNA (PCMdsRNA)
Complex, B/P = 7.0

To 4.0 ml. of a solution of $1.24 \times 10^{-4}$ mM
phosphorus ($OD_{260} = 0.895$) PCMdsRNA in .02M Tris, 0.1M
NaCl, pH 7.0 buffer were added 4.0 ml. of a solution
containing 0.57 mg./ml. of apramycin in the same buffer
brought to pH 7.0 with 0.1N HCl. The potency of the
apramycin sample was 0.92 mg. free base/mg. sample.

The final concentrations were $4.35 \times 10^{-4}$ mM
apramycin as free base and 6.21 $\times 10^{-5}$ mM PCMdsRNA
phosphorus which is a B/P of 7.0. The solution was
incubated 1 hour at room temperature to ensure complex
formation before use.

## Example 7

Neomycin-Poly I-Poly C Complex, B/P = 7.0

To 4.0 ml. of Poly I-Poly C in 0.02$\underline{M}$ Tris, 0.1$\underline{M}$ NaCl, pH 7.0 buffer ($OD_{260}$ = 0.64, concentration of phosphorus = 1.23 x $10^{-4}$ mM) were added 4.0 ml. of a solution containing 0.77 mg./ml. of neomycin sulfate in the same buffer brought to pH 7.0 with 0.1$\underline{N}$ HCl. The potency of the neomycin sulfate sample was 0.70 mg. free base/mg. sample.

The final concentrations were 4.35 x $10^{-4}$ mM neomycin as free base and 6.15 x $10^{-5}$ mM Poly I-Poly C phosphorus which is a B/P of 7.0. The solution was incubated for 1 hour at room temperature to ensure complex formation before use.

The activity of these complexes against the effects of tumor cell metastasis is demonstrated using Madison (M109) lung carcinoma, a highly refractory tumor system to conventional cytoreductive therapy [Marks et. al., Cancer Treatment Reports 61, 1459-1470 (1977)]. This carcinoma can be carried as a transplantable line in syngeneic BALB/C mice. The tumor line is available from the tumor bank at Mason Research Institute.

The interferon-inducing capacity of the complexes is demonstrated by a recognized procedure. In this procedure, dsRNA is dissolved in 0.15$\underline{M}$ saline 0.02$\underline{M}$ Tris buffer at a concentration suitable for administration, generally about 20 µg./ml. The dsRNA-tobramycin complex is prepared by addition to the dsRNA solution of tobramycin in an amount to produce the desired B/P ratio. The resulting mixture is incubated for about one hour at room temperature to effect optimal tobramycin-dsRNA binding.

Four mice are injected intraperitoneally (i.p.) with the solution (generally 0.5 ml.) containing a total of 10 µg. of dsRNA whether alone or as the tobramycin-dsRNA complex. Sixteen hours following injection, the mice are bled, and the blood is pooled and centrifuged to obtain the serum. Half-log dilutions of the serum are made using M199 medium, and the separate dilutions (2.5 ml.) are applied to confluent mouse L-cell cultures in Falcon 25 cm.$^2$ tissue culture flasks and allowed to incubate for 20 hours. The cultures then are inoculated with vesicular stomatitis virus in 2.5 ml. of M199 medium at a virus concentration producing 60-100 plaques per flask. After two hours viral adsorption, the fluid is removed, and M199 agar (65%) is applied and allowed to harden. After three days, the flasks are treated with 2% sodium acetate and 3.7% formaldehyde to fix the cells. The plaques are counted, and the amount of interferon is determined on the basis of plaque reduction relative to control plates lacking interferon. One unit of interferon is defined as the reciprocal of the dilution of the interferon solution (mouse serum) that provides 50% inhibition of virus plaque production. The amount of interferon then is calculated by the product of this determination and the total dilution.

The degradative effect of human serum dsRNase on the interferon-inducing capacity of dsRNA or dsRNA-tobramycin is determined by adding human serum to the solution of dsRNA or dsRNA-tobramycin complex. The amount of buffer is appropriately reduced to accomodate
the amount of added human serum and to maintain a
constant concentration of dsRNA. The resulting mixture
is incubated at 37°C. for one hour and then placed in
ice. The solutions are used within 15 minutes following removal from the 37°C. bath.

Table I following compares the actions of
dsRNA and a complex in inducing interferon following
treatment with human serum.

Table I

| Compound[a] | Human Serum[b] | Interferon, units |
| --- | --- | --- |
| tobramycin | Yes | 35 |
| dsRNA | No | 1050 |
| dsRNA + tobramycin | No | 3200 |
| dsRNA | Yes | 447 |
| dsRNA + tobramycin | Yes | 3470 |

    a. Dose -- 0.5 ml.; dsRNA concentration --
       20 µg./ml.; tobramycin concentration --
       14.5 µg./ml. (B/P=1).
    b. 1.2 µl./ml. of sample.

Table II following shows the effects on
interferon induction of varying (a) the B/P ratio
of tobramycin and dsRNA and (b) the concentration of
human serum.

Table II

| Compound[a] | B/P | Human serum, ml. per each 3 ml. sample | Interferon, units |
|---|---|---|---|
| Control (Buffer) | - | - | <31 |
| dsRNA | - | - | 3160 |
| dsRNA | - | 1.0 | <31 |
| dsRNA-tobramycin | 4 | - | 2570 |
| dsRNA-tobramycin | 4 | 1.0 | 1479 |
| dsRNA-tobramycin | 4 | 2.0 | 1230 |
| dsRNA-tobramycin | 4 | 2.5 | 346 |
| dsRNA-tobramycin | 6 | 1.0 | 2042 |
| dsRNA-tobramycin | 6 | 2.0 | 891 |
| dsRNA-tobramycin | 6 | 2.5 | 447 |

a.    Dose -- 0.5 ml.; dsRNA concentration --
20 µg./ml.

Table III shows the effect on interferon
induction after challenge with 83% human serum (2.5
ml. per 3.0 ml. sample) of various tobramycin-dsRNA
B/P ratios.

0080357

Table III

| Tobramycin-dsRNA, B/P | Interferon Induced, Units |
|:---:|:---:|
| 4 | 110 |
| 8 | 234 |
| 12 | 310 |
| 16 | 310 |
| 20 | 417 |
| 40 | 316 |
| 16[a] | 1667 |

a.   Run without human serum challenge

The activity of the complexes on macrophage activation has been determined according to a recognized procedure. In this procedure, peritoneal macrophages were harvested after 5 days from composition-treated mice by peritoneal lavage and purified by adherence on plastic. Approximately $4 \times 10^5$ macrophages in 16 mm. wells were overlaid with $4 \times 10^4$ P815 cells contained in 2 ml. of Roswell Park Memorial Institute 1640 Medium supplemented with 20% fetal calf serum. All cultures were maintained in a humidified, 5% $CO_2$-in-air incubator at 37°C., and cytotoxicity was assessed at 48 hours on the basis of viable cell counts in a hemocytometer. Triplicate cultures were maintained for each group; the mean cell count and standard error (S.E.) were calculated. Under these conditions, peritoneal macrophages from normal BALB/C mice treated with tris-buffered saline did not affect the growth of P815 target cells, as measured both by viable cell number and by DNA synthesis of the leukemia cells. The ratio of macrophages to target cells was approximately 10:1 at the beginning of each experiment. The percentage of growth inhibition of P815 cells due to composition-mediated macrophage activation was calculated by comparison to that of P815 cells grown in the presence of macrophages from buffer-treated animals. The results are provided in Table IV following:

Table IV

In Vivo Macrophage Activation Using
dsRNA-Tobramycin Complex

| Compound | Amount of Compound[a] | Amount of Human Serum | Percent Macrophage Cytotoxicity | |
|---|---|---|---|---|
| | | | Expt. 1 | Expt. 2 |
| dsRNA | 10 μg. | -- | 91 | 70 |
| dsRNA | 10 μg. | 0.002 ml. | 78 | 58 |
| dsRNA | 10 μg. | 0.42 ml. | 30 | 6 |
| dsRNA-tobramycin | 10 μg.+100 μg. | -- | 89 | 81 |
| dsRNA-tobramycin | 10 μg.+100 μg. | 0.42 ml. | 74 | 69 |
| Tobramycin | 100 μg. | -- | -1 | 0 |
| None | - | -- | 0 | 0 |

[a] A total of 0.5 ml. of incubation mixture was given i.p. 5 days prior to harvest of peritoneal macrophages. Dose given is the amount injected per mouse.

In conducting tumor metastasis studies, a subcutaneously-grown tumor is aseptically excised, minced using a scissors, and gently trypsinized at room temperature to obtain a single cell suspension. The cells are suspended in RPMI-1640 medium (M.A. Bioproducts, Walkersville, Maryland). Viable M109 cells are determined by trypan blue exclusion, and the cell concentration is determined using a hemocytometer. The cell concentration is adjusted to $1 \times 10^5$ viable cells per ml. of medium. The M109 cell-containing medium is injected into normal, male BALB/C mice in an amount providing the desired number of viable cells. Test compositions are administered intraperitoneally (i.p.) to randomized groups of ten mice each two days prior to tumor cell inoculation if the latter is intravenous (i.v.) and five days after tumor cell inoculation if the latter is subcutaneous (s.c.). Controls receive mock injections of 0.5 ml. carrier. Mortality is monitored to determine percent increased life span (ILS) relative to control, and the median survival time (MST) for each group is determined. Both are calculated from the time of tumor cell administration.

The following Table V provides results using complexes prepared from several aminoglycosides and P. chrysogenum - derived dsRNA. In the tests, $2 \times 10^4$ monodisperse M109 cells were administered i.v. two days after administering test compound.

0080357

Table V

| Compound (Day -2; i.p.) | Ionizable Nitrogens | Dose (µg) | MST (days) | ILS (%) |
|---|---|---|---|---|
| Control | - | | 32.5 | 0 |
| Tobramycin | 5 | 101.5 | 33.5 | 3 |
| Streptomycin | 3 | 167.5 | 33.0 | 1 |
| Neomycin | 6 | 133.0 | 34.0 | 5 |
| Apramycin | 5 | 130.5 | 34.5 | 6 |
| dsRNA | - | 10.0 | 39.0 | 20 |
| dsRNA-Tobramycin | 5 | 10+101.5 | 52.0 | 60 |
| dsRNA-Streptomycin | 3 | 10+167.5 | 38.5 | 18 |
| dsRNA-Neomycin | 6 | 10+133.0 | 50.0 | 54 |
| dsRNA-Apramycin | 5 | 10+130.5 | 41.0 | 26 |

Table VI following shows the effectiveness of compositions prepared from natural (P. chrysogenum-derived) and synthetic dsRNA.

Table VI

| Compound (Day -2; i.p.) | Dose, µg. | MST, days | ILS, % |
|---|---|---|---|
| Control | - | 47 | 0 |
| Tobramycin | 197 | 50 | 6 |
| dsRNA | 10.1 | 56 | 19 |
| dsRNA-Tobramycin | 10.1 + 197 | 66 | 40 |
| poly I:poly C | 10.1 | 65 | 38 |
| poly I:poly C-tobramycin | 10.1 + 197 | 77 | 64 |

X-5978                                    -22-

        Table VII demonstrates the effect of a variety
of B/P ratios of aminoglycoside and P. chrysogenum -
derived dsRNA on anti-tumor activity.

Table VII

| Compound[a] (Day -2; i.p.) | Dose, μg. | B/P | MST, days | ILS, % |
|---|---|---|---|---|
| Control | - | | 34.5 | 0.0 |
| dsRNA | 10 | | 37.5 | 8.7 |
| dsRNA-tobramycin | 10 + 101.5 | 7 | 45.5 | 31.9 |
| dsRNA-tobramycin | 10 + 14.5 | 1 | 42.5 | 23.2 |
| dsRNA-tobramycin | 10 + 1.4 | 0.1 | 35.0 | 1.4 |
| tobramycin | 101.5 | | 34.5 | 0.0 |
| tobramycin | 14.5 | | 37.5 | 8.7 |
| tobramycin | 1.4 | | 37.5 | 8.7 |

[a]Mice received 2 x 10$^4$ monodisperse M109 cells i.v. on
  Day 0.

-23-

Table VIII provides a dose response of the anti-tumor activity of a complex of aminoglycoside and P. chrysogenum - derived dsRNA having a B/P of 7.

Table VIII

| Compound[a] (Day -2; i.p.) | Dose, µg. | MST, days | ILS, % |
|---|---|---|---|
| Control | – | 32 | – |
| dsRNA | 40 | 34 | 7 |
| dsRNA | 10.45 | 37 | 14 |
| dsRNA | 2.5 | 34 | 7 |
| dsRNA-tobramycin | 40 + 405 | 45 | 41 |
| dsRNA-tobramycin | 10 + 101.5 | 48 | 50 |
| dsRNA-tobramycin | 2.5 + 25 | 35 | 9 |
| Tobramycin | 405 | 32 | 0 |

[a]Mice received $2 \times 10^4$ monodisperse M109 cells i.v. on Day 0.

CLAIMS

1. A composition for use in inhibiting the proliferation and migration of tumor cells which comprises a natural or synthetic double stranded RNA and an aminoglycoside having at least 5 ionizable nitrogens, said aminoglycoside and double stranded RNA being present in a molar ratio, aminoglycoside to double stranded RNA phosphorus, of at least 1:1.

2. The composition of claim 1 in which the aminoglycoside has 5 ionizable nitrogens.

3. The composition of claim 2 in which the aminoglycoside is tobramycin or apramycin.

4. The composition of claim 3 in which the aminoglycoside is tobramycin.

5. A complex comprising natural or synthetic double stranded RNA and tobramycin in a molar ratio of tobramycin to double stranded RNA phosphorus of at least about 1:1.

6. The composition of any of claims 1 to 4 or the complex of claim 5 in which the double stranded RNA is of natural origin.

7. The composition or the complex of claim 6 in which the source of the double stranded RNA is Penicillium chrysogenum.

8. The composition or the complex of any of claims 1 to 7 in which the molar ratio of aminoglycoside to double stranded RNA is from about 12:1 to about 1:1, preferably from about 9:1 to about 6:1.

9. A process for preparing a complex for use in inhibiting the proliferation and migration of tumor cells which comprises binding double stranded RNA and an aminoglycoside other than neomycin, said aminoglycoside having at least 5 ionizable nitrogens, in
amounts to obtain a molar ratio of aminoglycoside to
double stranded RNA phosphorus of at least 1:1.

10.   The process of claim 9 in which a solution
of lyophilized double stranded RNA in a sterilized
neutral buffer is added to a solution of aminoglycoside
in said buffer.

11.   The process of claim 9 or 10 in which the
combined double stranded RNA and aminoglycoside are
incubated for an hour to ensure optimal binding.

12.   The process of claim 9, 10 or 11 in which
the aminoglycoside has 5 ionizable nitrogens.

13.   The process of claim 12 in which the
aminoglycoside is tobramycin or apramycin.

14.   The process of claim 13 in which the
aminoglycoside is tobramycin.

15.   The process of any of claims 9 to 14 in
which the double stranded RNA is of natural origin.

16.   The process of claim 15 in which the
double stranded RNA source is _Penicillium_ _chrysogenum_.

17.   The process of any of claims 9 to 16 in
which the molar ratio of aminoglycoside to double
stranded RNA phosphorus is from about 12:1 to about
1:1, preferably from about 9:1 to 6:1.

CLAIMS

1. A process for preparing a complex for use in inhibiting the proliferation and migration of tumor cells which comprises binding double stranded RNA and an aminoglycoside other than neomycin, said aminoglycoside having at least 5 ionizable nitrogens, in amounts to obtain a molar ration of aminoglycoside to double stranded RNA phosphorus of at least 1:1.

2. The process of claim 1 in which a solution of lyophilized double stranded RNA in a sterilized neutral buffer is added to a solution of aminoglycoside in said buffer.

3. The process of claim 1 or 2 in which the combined double stranded RNA and aminoglycoside are incubated for an hour to ensure optimal binding.

4. The process of claim 1, 2 or 3 in which the aminoglycoside has 5 ionizable nitrogens.

5. The process of claim 4 in which the aminoglycoside is tobramycin or apramycin.

6. The process of claim 5 in which the aminoglycoside is tobramycin.

7. The process of any of claims 1 to 6 in which the double stranded RNA is of natural origin.

8. The process of claim 7 in which the double stranded RNA source is <u>Penicillium chrysogenum</u>.

9. The process of any of claims 1 to 8 in which the molar ratio of aminoglycoside to double stranded RNA phosphorus is from about 12:1 to about 1:1, preferably from about 9:1 to about 6:1.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | FR-A-2 227 867 (SOCIETTA FARMACEUTICI ITALIA) *Page 6, lines 1-17; claims 1-6* | 1-17 | C 07 H 21/04 A 61 K 31/71 |
| A | GB-A-1 410 013 (BEECHAM GROUP LIMITED) *Page 29, lines 3-14; claims 1-2* | 1-17 | |
| D,A | US-A-3 597 318 (ELISABETH SINCLAIR SUTHERLAND) *Column 2, lines 13-20* | 1-17 | |
| D,A | US-A-3 582 469 (JEROME BIRNBAUM) | 1-17 | |

TECHNICAL FIELDS SEARCHED (Int. Cl. ³)

C 07 H
A 61 K

The present search report has been drawn up for all claims

| Place of search THE HAGUE | Date of completion of the search 23-02-1983 | Examiner BRINKMANN C. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82